# EUROPÄISCHE PATENTSCHRIFT

(11) **EP 0 501 395 B1**
(45) Veröffentlichungstag und Bekanntmachung des Hinweises auf die Patenterteilung: **12.06.1996**
(21) Anmeldenummer: 92103111.8
(22) Anmeldetag: 25.02.1992
(51) Int. Cl.: C12Q 1/68, C12P 19/34

(54) **Verfahren zur Herstellung in vitro replizierbarer Nukleinsäuren**
Method for the production in vitro of replicatable nucleic acids
Procédé pour la production in vitro des acides nucléiques réplicables

(30) Priorität: 01.03.1991 DE 4106473
(43) Veröffentlichungstag der Anmeldung: 02.09.1992
(73) Patentinhaber: BOEHRINGER MANNHEIM GMBH, D-68298 Mannheim (DE)
(72) Erfinder: Rüger, Rüdiger, Dr. med., W-8124 Seeshaupt (DE); Kessler, Christoph, Dr. rer. nat., W-8021 Dorfen (DE)

(56) Entgegenhaltungen:
- WO-A-90/10064
- WO-A-91/01384
- WO-A-91/03573

## Beschreibung

Gegenstand der Erfindung sind ein Verfahren zur Herstellung in vitro replizierbarer Nukleinsäuren, Verfahren zur Vermehrung und zum Nachweis von Nukleinsäuren mittels einer Replikationsreaktion und Reagenzien, die zur Anwendung dieser Verfahren geeignet sind.

Die Vermehrung von Nukleinsäuren ist in jüngerer Zeit in erheblichem Umfang Gegenstand der Forschung geworden. Dies dürfte seinen Grund darin haben, daß Nukleinsäuren in der Natur praktisch nie in Reinform, sondern meist in Gemischen vorliegen. Insbesondere bei Nachweisverfahren, welche auf dem Nachweis von Nukleinsäuren in Proben beruhen, wie beispielsweise in der klinischen Diagnostik, der Lebensmitteldiagnostik und der Biotechnologie hat es sich als erforderlich erwiesen, die nachzuweisenden Nukleinsäuren in einem vorgeschalteten Schritt zu vermehren und ggf. anschließend nach konventionellen Methoden nachzuweisen. Auch bei der in-vivo Vermehrung von Nukleinsäuren ist ein vorgeschalteter in vitro Vermehrungsschritt von Vorteil.

Bei den Nukleinsäurevermehrungsverfahren kann man zwischen replikativen und transkriptiven Verfahren unterscheiden. Bei den auf Transkription basierenden Verfahren ist das Amplifikationsprodukt RNS. Ein solches Verfahren ist beispielsweise beschrieben in der EP-A-0 329 822. Ein Nachteil der transkriptiven Verfahren ist die Instabilität von RNS in den auf die Vermehrung von Nukleinsäuren folgende Reaktionen. Außerdem ist RNS nicht in jedem Fall direkt weiterverwendbar, wie dies z.B. beim direkten Klonieren der Amplifikationsprodukte notwendig ist.

Ein auf Replikation von genomischen Sequenzen eines RNS-Phagen beruhendes Verfahren zum Nachweis von Nukleinsäuren ist in der WO 87/06270 beschrieben. Dieses Verfahren beruht jedoch nicht auf einer Vermehrung der nachzuweisenden Nukleinsäure, sondern einer damit spezifisch hybridisierten Nukleinsäure. Dieses Verfahren ist daher nur zum Nachweis von Nukleinsäuren verwendbar und hat darüber hinaus eine hohe Background-Problematik.

In der WO 90/10064 ist ein Verfahren zur Herstellung von replizierbaren Nukleinsäuren beschrieben, welches darauf beruht, daß eine doppelsträngige nachzuweisende Nukleinsäure mit Restriktionsenzymen geschnitten wird und anschließend das so gebildete Fragment mit einer Nukleinsäure, die eine ori-Sequenz aus dem Phagen ø 29 enthält, ligiert wird. Dieses Verfahren hat den Nachteil, daß mindestens zwei Enzyme zur Herstellung der replizierbaren Nukleinsäure erforderlich sind, nämlich ein Restriktionsenzym und die Ligase. Die Reaktion wird noch komplexer, wenn von RNS ausgegangen werden soll.

Aufgabe der vorliegenden Erfindung war es daher, ein besonders einfaches Verfahren zur Herstellung von replizierbaren Nukleinsäuren bereitzustellen.

Gegenstand der Erfindung ist ein Verfahren zur Herstellung in vitro replizierbarer Nukleinsäuren, welches folgende Schritte umfaßt:
a) Bereitstellung einer einzelsträngigen Nukleinsäure A;
b) Hybridisierung der Nukleinsäure A mit einem Primer P1, welcher einen zu einem Teil der Nukleinsäure A komplementären Teil und eine Replikationsinitiationsstelle enthält;
c) Elongation des Primers P1 mit einer Polymerase unter Verwendung von Mononukleosidtriphosphaten zu einer Nukleinsäure B;
d) Entfernung von A aus dem Nukleinsäurehybrid aus A und B;
e) Hybridisierung der Nukleinsäure B mit einem Primer P2, welcher einen zu einem Teil der Nukleinsäure B komplementären Bereich und eine Replikationsinitiationsstelle enthält; und
f) Elongation des Primers P2 mit der Polymerase unter Verwendung von Mononukleosidtriphosphaten zu einer Nukleinsäure C.

Ebenfalls Gegenstand der Erfindung sind ein Verfahren zur Vermehrung von Nukleinsäuren oder Teilen davon, welches von diesem Verfahren Gebrauch macht und ein Verfahren zum Nachweis von Nukleinsäuren, welches das Vermehrungsverfahren einschließt. Ebenfalls Gegenstand der Erfindung ist ein Reagenzkit zur Herstellung in vitro replizierbarer Nukleinsäuren und zum Nachweis von Nukleinsäuren.

Im Folgenden wird als eine zu einer anderen Nukleinsäure im wesentlichen komplementäre Nukleinsäure eine Nukleinsäure bezeichnet, die mit der anderen Nukleinsäure hybridisieren kann, obgleich die Basenpaarung in einer oder mehreren Nukleotiden nicht der Regel nach Watson und Crick entspricht.

Als eine zu einer anderen Nukleinsäure im wesentlichen homologe Nukleinsäure wird eine Nukleinsäure bezeichnet, deren Nukleotidsequenz sich von der Nukleotidsequenz der anderen Nukleinsäure in einer oder mehreren Nukleotiden unterscheidet, die aber dennoch mit einer zu der anderen Nukleinsäure komplementären Nukleotidsequenz hybridisieren kann.

In dem erfindungsgemäßen Verfahren können als Nukleinsäuren A alle Arten von Nukleinsäuren eingesetzt werden. Dazu gehören sowohl RNS als auch DNS, beide in Einzel- oder Doppelstrangform, natürlichen wie synthetischen Ursprungs. Sie können eukaryonten (z.B. Viren, Zellen) aber auch prokaryonten (z.B. Phagen, Bakterien) Ursprungs sein. Wenn die Nukleinsäuren in doppelsträngiger Form vorliegen, müssen sie in Einzelstränge überführt werden. Dies kann auf konventionelle Weise geschehen, beispielsweise durch Erhitzen oder mit Hilfe geeigneter Reagentien.

Die Herkunft der Nukleinsäuren spielt keine Rolle. Bevorzugt können Nukleinsäuren in Lösungen, Suspensionen, aber auch fixiert an Festkörpern, oder in zellhaltigen Medien, Zellabstrichen, fixierten Zellen bzw. Gewebsschnitten, oder an isolierten Chromosomen benutzt werden. Besonders bevorzugt ist die Verwendung von gelösten Nukleinsäuren. Beispiele sind virale DNS, bakterielle RNS oder zelluläre genomische DNS. Das Medium, in dem die einzelsträngigen Nukleinsäuren A vorliegen, wird im Folgenden als Probemedium bezeichnet. Dieses Medium kann neben der Nukleinsäure A auch noch andere Bestandteile, insbesondere Nukleinsäuren, enthalten, die nicht in replizierbare Nukleinsäuren eingebaut und/oder vermehrt werden sollen.

Als template Nukleinsäure wird im folgenden eine Nukleinsäure verstanden, welche eine Nukleotidsequenzinformation beinhaltet, die in einem Nukleinsäuresyntheseschritt an eine neu gebildete Nukleinsäure weitergegeben wird. Eine dieser template Nukleinsäuren ist die dem erfindungsgemäßen Verfahren zugrundeliegende Nukleinsäure A.

Unter einer replizierbaren Nukleinsäure wird eine Nukleinsäure verstanden, die nach Zugabe eines Replikationssytems vervielfältigt (amplifiziert) werden kann, und welche mindestens eine ori-Region enthält.

In dieser Erfindung wird die ori-Region eines Replikationssystems über eine Reaktion eingebracht, die nur ein einziges Enzym erfordert. Insgesamt werden zur Durchführung des Verfahrens pro Nukleinsäure A mindestens zwei Startmoleküle benötigt, die im Folgenden Primer genannt werden. Unter einem Primer wird im Folgenden eine Nukleinsäure, insbesondere ein Oligonukleotid verstanden, welche einen zu einem Teil der Nukleinsäure A komplementären Teil aufweist und daher mit der Nukleinsäure A spezifisch hybridisieren kann. Der Primer enthält außerdem noch Nukleotidsequenzen, die nicht zu der Nukleinsäure A komplementär sind. Dazu gehören die ori-Sequenzen eines Replikationssystems. Außerdem kann der Primer weitere Nukleotide enthalten, beispielsweise solche, die eine Restriktionsenzymschnittstelle darstellen.

Die Nukleotidsequenz der Primer wird so gewählt, daß ihre 3'-Enden auf der Nukleinsäure A bevorzugt 1 bis 20000, besonders bevorzugt 100-8000 Nukleotide weit voneinander entfernt liegen.

Ori (origins of replication)-Sequenzen sind Replikationsinitiationsstellen, wie sie beispielsweise in B. Lewin, Genes IV, Verlag John Wiley 1990 beschrieben sind. Zu diesen ori-Sequenzen gehört jeweils ein Replikationssystem. Unter Replikationssystem werden die zur Replikation einer Nukleinsäure erforderlichen Reagenzien verstanden. Dazu gehört insbesondere ein Replikationsenzym, bevorzugt eine DNS-Polymerase, und gegebenenfalls Cofaktoren. Solche Replikationssysteme sind beispielsweise aus den Phagen PRD1, ø15, M29, Nf, GA-1, Cp1 und ø29 sowie Eukaryonten, wie Adenoviren, bekannt. Bevorzugt ist das Replikationssystem des Bacillus subtilis Phagen ø29. Hier haben sich die DNS-Polymerase p2 und das die Replikation initiierende Protein p3 als besonders vorteilhaft erwiesen.

Als Primer P1 wird erfindungsgemäß ein Primer benutzt, welcher neben einem zu einem Teil der Nukleinsäure A komplementären Teil eine Replikationsinitiationsstelle beinhaltet. Als Primer P2 wird erfindungsgemäß ein Primer benutzt, welcher einen zu einem Teil der Nukleinsäure A im wesentlichen homologen Teil und eine Replikationsinitiationsstelle enthält. Während der zur Nukleinsäure A komplementäre bzw. homologe Teil im 3'-Endbereich der Primer liegt, befindet sich die Replikationsinitiationsstelle bevorzugt im 5'-Bereich der Primer. Der zu einem Teil der Nukleinsäure A komplementäre bzw. homologe Teil eines Primers wird im Folgenden als templatespezifische Sequenz des Primers bezeichnet. Diese Sequenz ist bevorzugt 6 bis 50 Nukleotide lang. Im Gegensatz zu dem Vorgehen in der WO 90/10064 kann damit auf einfache Weise erreicht werden, daß spezifisch beliebige bestimmte Nukleinsäuren A oder Teile davon in replizierbare Nukleinsäuren umgewandelt werden. Die ori-Sequenz ist beispielsweise im bevorzugten ø29-System bis zu 280 Nukleotide bevorzugt 12 bis 80 Nukleotide, lang. Zu weiteren Details des Replikationssystems von ø29 und den zugehörigen ori-Sequenzen wird vollinhaltlich auf folgende Publikationen verwiesen:
DNS Replication and Mutagenesis, edts. Moses, Summers,
ASM, Washington DC, 1988;
Proc. Natl. Acad. Sci. USA 82, 6404 (1985);
Nucl. Acids Res. 16/13, 5895 (1988);
J. Biol. Chem. 264/15, 8935 (1989);
WO 90/10064;
Biochem. Biophys. Acta 95, 419.

Die Primer sind bevorzugt einzelsträngig.

Die Reaktion wird durch Hybridisierung der Nukleinsäure A mit Primer 1 und dessen Elongation mittels einer Polymerase und Monodesoxyribonukleosidtriphosphaten (dNTP) an A als template gestartet. Als Polymerase wird eine DNS-Polymerase (DNAP) eingesetzt. Je nachdem, ob DNS oder RNS als Nukleinsäure A vorliegt, wird eine DNS-abhängige Polymerase z.B. Kornberg-Polymerase, Klenow-Polymerase, T3-, T4- oder T7-DNS-Polymerase oder Taq-Polymerase, oder eine RNS-abhängige DNS-Polymerase, wie reverse Transkriptase (beispielsweise aus Avian Myelob-lastoses-Virus oder Moloney Murine Leukemea-Virus) eingesetzt. In dieser Reaktion wird ein Nukleinsäurestrang B gebildet, welcher am 5'-Ende eine ori-Sequenz enthält, und der am 3'-Ende zu A im wesentlichen komplementär ist.

Anschließend wird A aus dem Nukleinsäurehybrid aus A und B entfernt. Dies kann beispielsweise durch die Denaturierung dieses Doppelstranges oder Abbau von A geschehen.

Danach wird die Nukleinsäure B mit dem zweiten Primer P2, welcher zu einem neu gebildeten Teil des Stranges B im wesentlichen komplementär ist, hybridisiert und der Primer P2 analog zu Schritt c) unter Verwendung der DNS-Polymerase und Mononukleosidtriphosphaten an B als template-Nukleinsäure zu einem Nukleinsäurestrang C verlängert. Dieser enthält sowohl am 5'-Ende als auch am 3'-Ende eine ori-Sequenz. Ein Ende des somit gebildeten Nukleinsäurehybrids aus Strang B und Strang C besitzt nun eine doppelsträngige ori-Sequenz und kann somit direkt repliziert werden.

Bevorzugt wird das erfindungsgemäße Verfahren in der angegebenen Reihenfolge durchgeführt. Es kann jedoch in der Reihenfolge der Schritte a) bis f) in gewissem Maße variiert werden. Beispielsweise ist eine gleichzeitige Zugabe der beiden Primer zum Start der Reaktion möglich, insbesondere dann, wenn eine Hybridisierung der Primer mit sich selbst ausgeschlossen ist, z.B. wenn die Primer keine 3'-komplementären Nukleotide aufweisen. Dieses Vorgehen ist außerdem bevorzugt, wenn auch der zu Nukleinsäure A komplementäre Strang in der Probe einzelsträngig vorlag und ebenfalls zur Herstellung replizierbarer Nukleinsäuren verwendet werden soll.

Als DNS-Polymerase kann auch eine thermostabile DNS-Polymerase eingesetzt werden. Es ist dann nach einer Hitzedenaturierung (wie z.B. in Schritt d) möglich) kein weiteres Zupipettieren der Polymerase erforderlich. Die Denaturierung nach Synthese des Stranges B kann aber auch mit anderen Mitteln durchgeführt werden (z.B. alkalisch oder mittels chaotroper Salze).

Prinzipiell können mit dem erfindungsgemäßen Verfahren neben der Nukleinsäure A auch weitere Nukleinsäuren bzw. Teile davon mit unterschiedlicher Nukleotidsequenz, bevorzugt auch der zu A komplementäre Strang, in demselben Probemedium zur Herstellung von replizierbaren Nukleinsäuren verwendet werden. Voraussetzung ist, daß für jeden Nukleinsäureeinzelstrang die entsprechenden Primer vorhanden sind.

Soweit Details zu den einzelnen Reaktionsschritten nicht ausdrücklich aufgeführt sind, handelt es sich um im wesentlichen aus Standardwerken der Molekularbiologie bekannte Verfahrensschritte oder Reagenzien. Es wird hierzu insbesondere auf in Nucleic acid hybridisation, Herausgeber Hames, Higgins, IRL Press 1986, Current Protocols in Molecular Biology, Hrsg. Ausubel et al. Wiley & Son, 1987 und Molecular Cloning, Hrsg. Sambrook et al. CSH, 1989, beschriebene Details verwiesen. Dazu gehören insbesondere die bekannten Methoden zur Herstellung von markierten Nukleosidtriphosphaten, die chemische und enzymatische Synthese von modifizierten und unmodifizierten Oligonukleotiden, die Spaltung von Nukleinsäuren mittels Restriktionsenzymen, die Auswahl von Hybridisierungsbedingungen, durch welche eine Spezifität erreicht werden kann, sowie die Bildung von Nukleinsäuren aus Nukleosidtriphosphaten mit Hilfe von Polymerasen gegebenenfalls unter Verwendung von sogenannten Primern.

Ebenfalls Gegenstand der Erfindung ist ein Verfahren zur Vermehrung von Nukleinsäuren A oder Teilen davon, welches von dem oben beschriebenen Verfahren zur Herstellung in vitro replizierbarer Nukleinsäuren Gebrauch macht. Dazu schließt sich an das Verfahren zur Herstellung der Nukleinsäuren die Replikation dieser Nukleinsäuren an, die zu Nukleinsäuren führt, die wiederum replizierbar sind. Wie beschrieben, ist das Produkt des erfindungsgemäßen Herstellungsverfahrens ein Nukleinsäurehybrid, welches an einem Ende eine dopppelsträngige ori-Sequenz aufweist. Einer der Stränge (Strang C) ist an beiden Enden durch ori-Sequenzen begrenzt.

Dieser Strang dient als Matrize für die Synthese eines Gegenstrangs D gleicher Länge durch Replikationsstart an der doppelsträngigen ori-Sequenz. Dabei startet im Fall des ø29-Systems p2 mit dem Einbau eines p3-AMP-Moleküls gegenüber dem 3'-T am doppelsträngigen ori-Ende des B-C-Hybrids und synthetisiert den komplementären Strang unter Verdrängung des Strangs C. Das doppelsträngige Molekül, welches nach dieser Reaktion vorliegt, entspricht einem linearen Replicon, dessen beiden Ende Origin-spezifisch sind. Die folgende Replikationsreaktion führt abhängig von Prozessivität und Geschwindigkeit der verwendeten Polymerase zu einer Amplifikation des Moleküls.

Dazu wird der gebildete Komplex aus Nukleinsäure B und C mit dem Replikationssystem umgesetzt, das unter geeigneten Bedingungen in der Lage ist, über eine in vitro Replikationsreaktion, die eine enzymatische Strangtrennung beinhaltet, zu Nukleinsäuren, die an ihren Enden jeweils eine ori-Sequenz beinhalten, unter Verwendung geeigneter Co-Faktoren eine komplementäre Nukleinsäure zu bilden. Diese Nukleinsäure steht dann wiederum zur Replikation bereit.

Bevorzugte Proteine des Replikationssystems sind solche mit DNS-Polymeraseaktivität, die keinen weiteren Nukleinsäure-Primerzusatz benötigen. Ein solches Enzym, welches in-vitro eine Replikationsreaktion initiieren kann, ist bei Verwendung von ø29 ori-Sequenzen das Enzym p2 des Phagen ø29 zusammen mit p3 und dATP. Dieses Enzym hat sowohl DNS-Polymerase- als auch Strangaufwindungsaktivitäten. Ein separater Denaturierungsschritt zwischen den einzelnen Vermehrungsschritten kann wegen der enzymatischen Strangtrennung während der Vermehrungsreaktion entfallen. Das erfindungsgemäße Verfahren hat daher den Vorteil, daß es kontinuierlich und schnell verlaufen kann. Außerdem kann es ohne größere Änderung der Reaktionstemperaturen und ohne Zugabe weiterer Reagenzien ablaufen. Da die neu gebildeten Replikate immer wieder in die Replikationsreaktion eingesetzt werden können, wird im Idealfall ein exponentieller Anstieg der neu gebildeten Nukleinsäuren C und D erreicht. Die gebildeten Nukleinsäuren C bzw. D enthalten neben den ori-Sequenzen mindestens eine Nukleotidsequenz die im wesentlichen komplementär oder homolog zu der Nukleinsäure A oder einem Teil davon ist.

Wenn die gewünschte Anzahl von Nukleinsäuren C und D gebildet ist, kann die Reaktion beendet werden. Dies geschieht bevorzugt durch Zugabe eines Stoppreagenzes, beispielsweise EDTA. Die zur Herstellung der Nukleinsäuren erforderliche Reaktionszeit wird gegenüber den Verfahren des Standes der Technik durch das erfindungsgemäße Verfahren stark herabgesetzt. Sie hängt wie bei den bekannten Verfahren unter anderem von der Länge der gebildeten Nukleinsäuren ab. Für weitere Details zur Vermehrung replizierbarer Nukleinsäuren wird auch auf WO 90/10064 verwiesen.

Die Nukleinsäuren C und D bzw. aus ihnen gebildete Komplexe können Gegenstand weiterer Reaktionen sein. Beispielsweise können aus ihnen gebildete Doppelstränge mit Restriktionsenzymen geschnitten werden. In diesem Fall werden im Verfahren zur Herstellung der replizierbaren Nukleinsäuren bevorzugt Primer eingesetzt, die in ihrem doppelsträngigen Bereich eine Schnittstelle für das Restriktionsenzym aufweisen.

Die Primer können auch eine Sequenz zur Hybridisierung mit M13- universellen Sequenzierungsprimern und/oder M13-universellen reverse Sequenzierungsprimern enthalten.

Die Nukleinsäuren C und D können auch Gegenstand von Reaktionen zur Einführung von radioaktiven oder nicht radioaktiven Markierungen sein, sodaß die Nukleinsäuren C und D leicht nachgewiesen werden können.

Ob die gewünschte Anzahl von Nukleinsäuren C und D gebildet wurde, kann durch bekannte Methoden ermittelt werden, beispielsweise durch Gelchromatographie.

Das erfindungsgemäße Verfahren hat verschiedene Vorteile: Die Reaktionsequenz kann, beispielsweise bei Verwendung des Replikationssystems des Phagen ø 29, bei einer Temperatur durchgeführt werden; hohe Temperaturen zur Denaturierung sind nicht erforderlich. Die in vitro-Replikation funktioniert mit langen DNS-Fragmenten bis zu 19 kb (mit ø 29 DNS) und mindestens bis zu 7 kb (M13-Genom) mit heterologer DNS. Im Vergleich dazu ist die Größe der nach EP-A-0201184 amplifizierbaren Sequenzen maximal 3-4 kb.

Die Amplifikationsrate ist theoretisch höher bzw. die Vermehrung der Nukleinsäuren schneller als bei anderen Target-Amplifikationsmethoden. Die Methode ist, da bei 30^{o}C - 37^{o}C durchführbar, zur Amplifikation in in situ-Hybridisierungsexperimenten, beispielsweise in Zellabstrichen, fixierten Zellen bzw. Gewebsschnitten, oder an isolierten Chromosomen, anwendbar. Die relativ kurzen Primer diffundieren in Gewebe ähnlich gut wie Oligonukleotide.

Ein Vorteil des erfindungsgemäßen Verfahrens ist, daß die Replikation nicht von der Sekundärstruktur des Gesamtkomplexes abhängig ist. Daher ist es möglich, auch lange Nukleinsäuren herzustellen. Außerdem müssen nicht dNTPs und NTPs eingesetzt werden, es genügt die Verwendung von dNTPs.

Durch Einführung von Restriktionsenzym-Schnittstellen in den Primern zwischen der templatespezifischen Region und der für das Replikationssystem spezifischen Region können die entstandenen Produkte z.B. direkt kloniert bzw.sequenziert werden oder als Probe von den Adaptor-Sequenzen freigeschnitten werden. Dies ist mit den Produkten aus Transkriptions-Amplifikationsverfahren nicht möglich.

Das erfindungsgemäße Verfahren zur Vermehrung von Nukleinsäuren ist außerordentlich zeitsparend und äußerst empfindlich. Inklusive Primerhybridisierung, Elongation und Replikation ergibt sich für ein 100-Nukleotide enthaltendes Teilstück der Nukleinsäure A unter Verwendung des ø 29-in-vitro Replikationssystems ein Zeitbedarf bis zu ca. 1 h mit einer theoretischen Amplifikationsrate von 2⁶⁹²; Verfahren des Standes der Technik würden hierfür im günstigsten Fall 2-4 h benötigen. Das erfindungsgemäße Verfahren kann bevorzugt als Eintopfreaktion geführt werden, bevorzugt über aufeinanderfolgende Zugabe zuerst der Komponenten der Schritte a - f und dann der Komponenten für die in-vitro Replikation.

Die in der Vermehrungsreaktion entstehenden Nukleinsäuren C und D haben bevorzugt die gleiche Länge. Dies ist vorteilhaft, da eine homogene Nukleinsäurepopulation gleichmäßigere Bedingungen für deren Nachweis, beispielsweise bei Hybridisierungen, ermöglicht.

Die Nukleinsäuren B, C und D sind DNS. Dies hat den Vorteil, daß die aus ihnen gebildeten Nukleinsäuren C und D wieder als Templat für Bildung von D und C in der gleichen Reaktion ohne zwischengeschaltete separate Reaktionsschritte benutzt werden können. Dadurch, daß die neu gebildeten Nukleinsäuren C und D bereits zwei für das Replikationssystem spezifische Sequenzen aufweisen, ist die erneute Zugabe von Primern überflüssig.

Das erfindungsgemäße Vermehrungsverfahren kann zur Herstellung einer Vielzahl von Kopien von Nukleinsäuren A oder Teilen von ihr bzw. dazu komplementären Nukleinsäuren verwendet werden. Dies ist insbesondere in der molekularbiologischen und genetischen Grundlagenforschung, in der klinischen Diagnostik und der Biotechnologie, oder zum Studium von Struktur und Funktionen seltener Gene wichtig.

Gegenstand der vorliegenden Erfindung ist auch ein Verfahren zum Nachweis von Nukleinsäuren A, welches das oben genannte Verfahren zur Vermehrung von Nukleinsäuren einschließt.

Dazu wird eine Probe, von der vermutet wird, daß sie die nachzuweisende Nukleinsäure enthält, dem oben genannten Verfahren zur Herstellung von Nukleinsäuren ausgesetzt, wobei die nachzuweisende Nukleinsäure genauso behandelt wird, wie oben für die Nukleinsäure A beschrieben.

Die Nukleinsäuren B, C und D können dann mit Hilfe von modifizierten oder nicht modifizierten Nukleosidtriphosphaten hergestellt werden. Bevorzugt werden modifizierte Nukleosidtriphosphate eingesetzt. Solche modifizierten Nukleosidtriphosphate sind bekannt. Die Modifikation kann beispielsweise im Ersatz eines oder mehrerer Reste des Nukleosidtriphosphates durch einen radioaktiven, fluoreszierenden, farbigen, immunreaktiven, biospezifisch bindefähigen oder chemisch reaktiven Rest bestehen. Geeignete immunreaktive Reste sind beispielsweise Haptene, wie Digoxigenin oder Sulfonsäurereste. Biospezifisch bindefähige Reste sind beispielsweise Vitamine, wie Biotin, und ein chemisch reaktiver Rest ist beispielsweise eine zusätzliche Aminogruppe oder Sulfhydrylgruppe, die evtl. über eine Brücke an dem Nukleosidtriphosphat angebracht ist. Im Fall des Einsatzes modifizierter Nukleosidtriphosphate wird zum Nachweis der Anwesenheit oder Menge der Nukleinsäure A einfach die Menge oder die Anwesenheit der Nukleinsäuren B, C bzw. D oder auch nur von C und D über die eingebaute Modifizierung nach Abtrennung nicht umgesetzter Nukleosidtriphosphate bestimmt. Besonders bevorzugt findet die Markierung nur während der in-vitro Replikation statt. Es sind dann C und D nachweisbar markiert. Das Vorgehen über Inkorporierung bereits modifizierter Nukleotide ist besonders vorteilhaft, da dann übliche weitere Hybridisierungsschritte mit nachweisbar markierten Nukleinsäuresonden oder Elongationsreaktionen entfallen können.

Verfahren zum Nachweis von Nukleinsäuren, in die modifizierte Nukleotidphosphate eingebaut sind, sind bekannt, im Fall der Haptenmarkierung beispielsweise aus der EP-A-0324468, im Fall der Biotinmarkierung aus der DE-A-2915082.

Bevorzugt werden die hybridisierten Nukleinsäuren dazu an Molekularsieben oder Affinitätsmaterialien, die einen Rest der Nukleinsäure, beispielsweise p3, erkennen und binden, von nicht umgesetzten Komponenten getrennt. Anschließend wird die Menge an Markierung bestimmt.

Es ist jedoch auch möglich, insbesondere bei Einsatz nicht modifizierter Nukleosidtriphosphate, die gebildeten Nukleinsäuren C bzw. D durch Hybridisierung mit einer wenigstens zum Teil dazu im wesentlichen komplementären modifizierten Nukleinsäure nachzuweisen. Diese Nukleinsäuren können nachweisbar sein oder gemacht werden. Auch solche Verfahren sind bekannt. Beispielhaft beschrieben ist ein solches Verfahren in der US-A-4358535 oder der EP-A-0192168.
Ein besonders bevorzugtes Verfahren zum Nachweis von Nukleinsäuren A ergibt sich, wenn in der Replikationsreaktion nachweisbar modifizierte und damit markierte Monodesoxyribonukleosidtriphosphate eingesetzt werden und nach der Replikation die gebildeten Nukleinsäuren mit einer für die Nukleinsäuren A templatespezifischen Nukleinsäuresonde, meist einem Oligonukleotid, hybridisiert werden, wobei die Sonde eine oder mehr immobilisierbare Gruppen enthält. Danach wird das Hybrid aus nachweisbar markiertem Replikat und immobilisierbarer Sonde mit einer festen Phase in Kontakt gebracht, die die immobilisierbare Gruppe spezifisch binden kann. Dann wird die feste von der flüssigen Phase getrennt, gegebenenfalls gewaschen und die gebundene nachweisbare Markierung bestimmt. Für weitere Details wird auf die DE-A-4041608 verwiesen. Ein Vorteil dieses Vorgehens ist, daß eine vollständige und damit aufwendige Abtrennung der überschüssigen und nicht hybridisierten Sonden nicht unbedingt erforderlich ist.

Ganz besonders bevorzugt ist dieses Verfahren, wenn die Replikate vor Umsetzung mit der Sonde nicht-thermisch denaturiert werden, z.B. alkalisch, und die Sonde anschließend einzelsträngig in einer Lösung, die sowohl Reagenzien, die die Denaturierungsbedingungen beseitigen (z.B. eine Säure), als auch Reagenzien, die die Hybridisierung begünstigen (z.B. Formamid), enthält, zugesetzt wird.

Das erfindungsgemäße Verfahren zur Vermehrung von Nukleinsäuren ist wegen des möglichen Einbaus modifizierter Nukleosidtriphosphate auch hervorragend als Verfahren zur Herstellung modifizierter Nukleinsäuren, insbesondere modifizierter DNS geeignet. Solche modifizierten Nukleinsäuren finden als sogenannte Probes in der DNS-Diagnostik Verwendung. Ist die Modifizierung eine nachweisbare Gruppe oder eine Gruppe, die in eine nachweisbare Gruppe umgewandelt werden kann, so erhält man Nukleinsäuren, wie sie als Detektionsprobe beispielsweise in der US-A-4358535 eingesetzt werden. Ist die Modifizierung eine bindefähige Gruppe, beispielsweise eine immunreaktive Gruppe oder Biotin, so erhält man Nukleinsäuren, wie sie in der EP-A-0097373 beschrieben sind. Sie sind als Fangprobe beispielsweise des in der EP-A-0139489 Verfahrens einsetzbar.

Das erfindungsgemäße Verfahren zum Nachweis von Nukleinsäuren vereinigt die Vorteile des Verfahrens zur Vermehrung von Nukleinsäuren bzw. Teilen davon mit denen der Markierung während der Vermehrung.
Mit diesem Verfahren können sowohl DNS als auch RNS nachgewiesen werden. Im Falle von RNS ist der Nachweis von rRNS bevorzugt, da hier besonders hohe Empfindlichkeiten bzw. auch geringe Reaktionszeiten zu erwarten sind. Dadurch wird beispielsweise Speziesdiagnostik möglich. Diese Speziesdiagnostik ist aber auch möglich über bakterienspezifische Gene wie Toxingene oder Pathogenitätsfaktoren.

Das erfindungsgemäße Verfahren kann auch zur Mutagenese über Mutageneseadaptoren oder über Primer, die im hybridisierenden einzelsträngigen Bereich mindestens einen mismatch haben, oder zum Nachweis von Mutationen über Primer, die im hybridisierenden einzelsträngigen Bereich mindestens einen mismatch haben, verwendet werden.

Eine besonders bevorzugte Ausführungsform des erfindungsgemäßen Verfahrens zur Herstellung von Nukleinsäuren wird durch die in-vitro Verwendung des Replikationssystems des Phagen ø 29 zur Bildung der Nukleinsäure C bzw. D aus dem Komplex der Nukleinsäuren A und B möglich. Dieses Replikationssystem ist beispielsweise in Biochim. Biophys. Act 951 (1988) 417-424 beschrieben.

In einer Ausführungsform der erfindungsgemäßen Verfahren wird die die Nukleinsäure A enthaltende Probe alkalisch denaturiert. Danach wird mit Zugabe des Primers 1 und gewünschtenfalls auch Primer 2 in einer angesäuerten Lösung neutralisiert und anschließend die Polymerase zusammen mit den Mononukleotiden zugegeben. Nach Inkubation wird erneut alkalisch denaturiert, mit einer angesäuerten Lösung neutralisiert,
Polymerase/Nukleotidgemisch zugegeben und inkubiert. Nach dieser Inkubation kann mit Zugabe des Replikationssystems die Amplifikationsreaktion gestartet werden. Eine 2 bis 15 min lange Vorhybridisierung vor der Elongationsreaktion der Primer ist möglich, sowohl vor der ersten als auch der zweiten Elongationsreaktion der Startreaktion. Von Vorteil dieser Variante ist die isotherme Gesamtreaktion. Es muß allerdings eine erhöhte Zahl von Pipettierschritten durchgeführt werden.

Die Reaktionsschritte des erfindungsgemäßen Vermehrungsverfahrens werden bei einer Temperatur zwischen 25 und 45^{o}C bei nicht-thermischer Denaturierung, bzw. 25-98C bei thermischer Denaturierung durchgeführt. Die Temperatur sollte sich insbesondere an der optimalen Temperatur der Polymerase orientieren (z.B. 37^{OC} bei Klenow oder 70^{o}C bei Taq-Polymerase).

Folgende Bedingungen haben sich für die Durchführung der Schritte a - f als vorteilhaft erwiesen:
Volumen: 20-50 µl; Puffersubstanzen: bevorzugt TrisHCl, pH 7,2-8,6 (bei 20°C), abhängig von verwendeter Polymerase; Salze: bevorzugt MgCl₂, 1,5 mM-15 mM (abhängig von verwendeter Polymerase KCl: 0-100 mM, vorteilhaft 20-100 mM bei Verwendung von Taq-DNAP;
- Hilfsmittel:: wie z. B. BSA, Dithiotreitol oder Dithioerythrit werden entsprechend der verwendeten Polymerase zugegeben, wie im allgemeinen dem Fachmann bekannt.
(Beispiel Klenow: Dithioerythrit, 50 µM-200 µM, BSA, 0,1-0,4 mg/Volumen);

Nukleosidtriphosphate: Entweder wird die Gesamtmenge sofort eingesetzt oder es wird erst eine für die Bildung des Startmoleküls notwendige Menge und vor Start der Replikation der Rest zugegeben. Dies ist besonders sinnvoll, wenn das Amplifikationsprodukt markiert werden soll.
Konzentration: 25-500 µM;
Polymerasen: 1-10 U (abhängig von Polymerase);
Inkubation:
1 min bis 30 min/Einzelschritt der Startreaktion (von template Nukleinsäure und verwendeter Polymerase abhängig).

Replikation: Volumen 20-200 µl; p3: 20-400 ng; p2: 2-300 ng; Puffer: Tris pH 7.5, 20-100 mM, Salze MgCl₂: 2-15 mM, (NH₄)₂SO₄: 0-50 mM, Hilfsstoffe: Spermidin 1-5 mM.
Inkubationszeit 15 min (für sehr kurze Regionen) bis 90 min (für lange Regionen). Nukleotide: wie oben jedoch alternativ: z.B. ³²P-dNTPs, Biotin-dNTPs oder Digoxin- oder Digoxigenin-dNTPs.

Zugabe von 50-500 mM NaCl oder KCl kann die Reaktion begünstigen.

Die angegebenen Werte dienen als Richtwerte. Es ist selbstverständlich, daß ein Fachmann Abwandlungen vornehmen kann.

Das erfindungsgemäße Nachweisverfahren kann theoretisch bis zur Detektion vollständig in einer Lösung ohne Abtrennung irgendwelcher Komponenten geführt werden. Erst dann ist eine Abtrennung überschüssigen Markierungsmittels erforderlich.

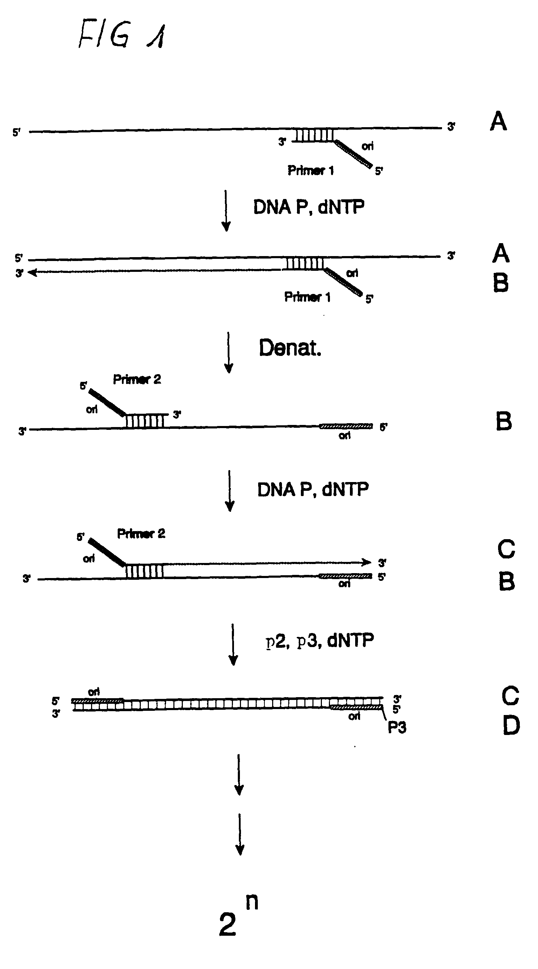

Fig. 1 zeigt schematisch die Reaktionsführung des erfindungsgemäßen Verfahrens zur Herstellung replizierbarer Nukleinsäuren und auch die weiteren Schritte zur Vermehrung der Nukleinsäuren.

Die folgenden Beispiele erläutern die Erfindung näher:

### Beispiel 1

Hepatitis B-Virus (HBV)-spezifische Sequenzen sollen amplifiziert werden. Dazu wird rekombinante HBV-DNS als Nukleinsäure A verwendet (siehe z.B. EP-B-0013828). Die Region zwischen Nukleotid-Position 1278 und 1571 wird amplifiziert unter Verwendung von Primern mit HBV-spezifischen Target-Sequenzen zwischen 1278 und 1297 und 1403 und 1422. Primer 1 besteht in 3'-5'-Richtung aus der HBV-spezifischen Sequenz von 1403 bis 1422 und daran anschließend 59 nt von der rechten ø29-ori-Sequenz (3'-5'-Orientierung). Der Primer 2 enthält in 3'-5'-Richtung die Nukleotidposition 1297 bis 1278 aus HBV und anschließend 46 nt von der linken ø29-ori-Sequenz (3'-5'-Orientierung).

HBV Nukleinsäure (0,5 µg-0,5 fg) wird 2 min bei 95°C denaturiert. Auf Eis werden TrisHCl, pH 7,2, 50 mM; MgCl₂, 10 mM; Dithioerythrit, 0,1 mM; Rinderserumalbumin, 0,2 mg/ml; Primer 1, 250 nM; dATP, 25 µM; dCTP, 25 µM; dGTP, 25 µM; dTTP, 25 µM und Klenow-DNS-Polymerase, 2 U zugegeben, so daß in einem Reaktionsvolumen von 20 µl die angegebenen Endkonzentrationen erhalten werden. Das Gemisch wird 15 min bei 37°C inkubiert. Danach wird erneut 2 min 95°C zur Denaturierung erhitzt. 2 U Klenow und die gleiche Menge an Primer 2 werden in 5 µl TrisHCl, pH 7,2, 50 mM; MgCl₂, 10 mM; Dithioerythrit, 0,1 mM;
Rinderserumalbumin, 0,2 mg/ml; dATP, 25 µM; dCTP, 25 µM; dGTP, 25 µM; dTTP, 25 µM zugegeben, und es wird erneut 15 min bei 37°C inkubiert.

Zur Inititation der Replikation (z. B. J. Biol. Chem., 1989, 264/15, 8935-8940) werden 150 ng des terminalen Proteins p3 von ø29 und 80 ng der ø29-DNS-Polymerase (p2) in 25 µl TrisHCl, 50 mM; MgCl₂, 10 mM;
Dithiotreitol, 2 mM; Spermidin, 2 mM; (NH₄)₂SO₄, 40 mM; dATP, 275 µM; dCTP, 275 µM; dGTP, 275 µM; dTTP, 275 µM zugegeben (Endvolumen 50 µl) und 30 min bei 37°C inkubiert. Die Reaktion wird mit 10 mM EDTA und 0,1 % SDS gestoppt. Die Reaktionsprodukte werden in 0,8 % Agarose-Gel aufgetrennt und durch Ethidium-bromid-Interkalation im UV-Licht sichtbar gemacht.

### Beispiel 2

Die selbe Region des in Beispiel 1 verwendeten rekombinanten HBV-Plasmids soll amplifiziert werden.

Das HBV-Plasmid (0,5 µg-0,5 fg) wird 2 min bei 95°C denaturiert. Auf Eis werden TrisHCl, pH 8,3, 50 mM; MgCl₂, 1,5 mM; KCl, 50 mM; Gelatine, 100 µg/ml; Primer 1, 250 nM; Primer 2, 250 nM; dATP, 150 µM; dCTP, 150 µM; dGTP, 150 µM; dTTP, 150 µM; und Taq-DNS-Polymerase, 2,5 U zugegeben, wobei in einem Endvolumen von 25 µl die angegebenen Endkonzentrationen erhalten werden. Es wird 10 min bei 70°C inkubiert, erneut 2 min bei 95°C denaturiert und der Inkubationsschritt bei 70°C nochmals durchgeführt. Danach wird das Gemisch auf 30°C abgekühlt. Zur Initiation der Replikation werden 150 ng des terminalen Proteins von ø29 (p3) und 80 ng der ø29-DNS-Polymerase (p2) in 25 µl TrisHCl, pH 7,0, 50 mM; MgCl₂, 18,5 mM; Spermidin, 2 mM; (NH₄)₂SO₄, 20 mM; zugefügt und 30 min bei 30°C inkubiert. Die Reaktion wird mit 10 mM EDTA und 0,1 % SDS gestoppt. Die Detektion der Amplifikationsprodukte wird wie in Beispiel 1 durchgeführt.

### Bezugszeichenliste

- A: zu replizierende Nukleinsäure in der Probe
- P1: Primer 1
- P2: Primer 2
- B: zu A zumindest teilweise komplementäre Nukleinsäure
- C: zu B zumindest teilweise komplementäre Nukleinsäure
- D: zu C komplementäre Nukleinsäure
- ori: Origin of replication, Replikationsinitiationsstelle
- DNA P: DNS-Polymerase
- dNTP: Monodesoxyribonukleosidtriphosphate
- p²,p³: Proteine des Replikationssystems des Phagen ø 29

## Patentansprüche

1. Verfahren zur Herstellung in vitro replizierbarer Nukleinsäuren aus einer template Nukleinsäure A, welches folgende Schritte umfaßt:
a) Bereitstellung einer einzelsträngigen Nukleinsäure A;
b) Hybridisierung der Nukleinsäure A mit einem Primer P1, welcher einen zu einem Teil der Nukleinsäure A komplementären Teil und eine Replikationsinitationsstelle enthält;
c) Elongation des Primers P1 mit einer Polymerase unter Verwendung von Mononukleosidtriphosphaten zu einer Nukleinsäure B;
d) Entfernung von A aus dem Nukleinsäurehybrid aus A und B;
e) Hybridisierung der Nukleinsäure B mit einem Primer P2, welcher einen zu einem Teil der Nukleinsäure B komplementären Bereich und eine Replikationsinitiationsstelle enthält und
f) Elongation des Primers P2 mit der Polymerase unter Verwendung von Mononukleosidtriphosphaten zu einer Nukleinsäure C.

2. Verfahren gemäß Anspruch 1, dadurch gekennzeichnet, daß
auch Primer P2 vor Elongation des Primers P1 zu der Probe, die A enthält, zugegeben wird.

3. Verfahren zur Vermehrung von Nukleinsäuren A oder Teilen davon, welches folgende Schritte umfaßt:
a) Bereitstellung einer einzelsträngigen Nukleinsäure A;
b) Hybridisierung der Nukleinsäure A mit einem Primer P1, welcher einen zu einem Teil der Nukleinsäure A komplementären Teil und eine Replikationsinitationsstelle enthält;
c) Elongation des Primers P1 mit einer Polymerase unter Verwendung von Mononukleosidtriphosphaten zu einer Nukleinsäure B;
d) Entfernung von A aus dem Nukleinsäurehybrid aus A und B;
e) Hybridisierung der Nukleinsäure B mit einem Primer P2, welcher ein zu einem Teil der Nukleinsäure B komplementären Bereich und eine Replikationsinitiationsstelle enthält;
f) Elongation des Primers P2 mit der Polymerase unter Verwendung von Mononukleosidtriphosphaten zu einer Nukleinsäure C; und
g) in-vitro-Replikation.

4. Verfahren zum Nachweis einer Nukleinsäure A, welches folgende Schritte umfaßt:
a) Herstellung einer replizierbaren Nukleinsäure nach dem Verfahren gemäß Anspruch 1
b) in-vitro-Replikation
c) Nachweis der durch die Replikation gebildeten Nukleinsäuren.

5. Verfahren nach Anspruch 4, dadurch gekennzeichnet, daß die in-vitro-Replikation unter Verwendung von nachweisbar oder/und immobilisierbar markierten Mononukleosidtriphosphaten durchgeführt wird.

6. Verfahren nach Anspruch 5, dadurch gekennzeichnet, daß ein nachweisbar markiertes Mononukleosidtriphosphat eingesetzt wird, die durch die Replikation gebildeten markierten Nukleinsäuren mit einer immobilisierbar markierten Nukleinsäuresonde hybridisiert werden und das gebildete Hybrid nach Bindung an eine feste Phase nachgewiesen wird.

7. Reagenzkit zur Herstellung in vitro replizierbarer Nukleinsäuren aus einer template Nukleinsäure A durch ein Verfahren gemäß Anspruch 1, enthaltend in getrennten Behältern:
- zwei Primer P1 und P2, welche jeweils Nukleotidsequenzen beinhalten, die für A templatespezifisch sind und die außerdem jeweils eine Replikationsinitiationsstelle enthalten,
- eine Polymerase, die in der Lage ist, einen Primer, welcher mit einer Nukleinsäure hybridisiert ist, mit Monodesoxyribonukleosidtriphosphaten zu verlängern,
- Monodesoxyribonukleosidtriphosphate.

8. Reagenzkit zur Vermehrung von Nukleinsäuren A oder Teilen davon durch ein Verfahren gemäß Anspruch 3, enthaltend in getrennten Behältern:
- zwei Primer P1 und P2, welche jeweils Nukleotidsequenzen beinhalten, die für A templatespezifisch sind und die außerdem eine Replikationsinitiationsstelle enthalten;
- eine Polymerase, die in der Lage ist, einen Primer, der mit einer Nukleinsäure hybridisiert ist, mit Monodesoxyribonukleosidtriphosphaten zu verlängern;
- ein in-vitro Replikationssystem;
- Monodesoxyribonukleosidtriphosphate.

9. Reagenzkit zum Nachweis von Nukleinsäuren A durch ein Verfahren gemäß Anspruch 4, enthaltend in getrennten Behältern:
- zwei Primer P1 und P2, welche jeweils Nukleotidsequenzen beinhalten, die für A template-spezifisch sind und die außerdem eine Replikationsinitiationsstelle enthalten;
- eine Polymerase, die in der Lage ist, einen Primer, der mit einer Nukleinsäure hybridisiert ist, mit Monodesoxyribonukleosidtriphosphaten zu verlängern;
- ein in-vitro Replikationssystem;
- Monodesoxyribonukleosidtriphosphate;
- eine für Nukleinsäure A spezifische, immobilisierbar markierte Nukleinsäuresonde.

10. Reagenzkit gemäß einem der Ansprüche 7 bis 9, dadurch gekennzeichnet, daß mindestens eines der Monodesoxyribonukleosidtriphosphate nachweisbar markiert ist.

## Claims

1. Process for the production of nucleic acids which are replicable in vitro from a template nucleic acid A which comprises the following steps:
a) provision of a single-stranded nucleic acid A;
b) hybridization of the nucleic acid A with a primer P1 which contains a part which is complementary to a part of the nucleic acid A and a replication initiation site;
c) elongation of the primer P1 with a polymerase using mononucleoside triphosphates to form a nucleic acid B;
d) removal of A from the nucleic acid hybrid of A and B
e) hybridization of the nucleic acid B with a primer P2 which contains a region which is complementary to a part of the nucleic acid B and a replication initiation site; and
f) elongation of the primer P2 with polymerase using mononucleoside triphosphates to form a nucleic acid C.

2. Process as claimed in claim 1, wherein primer P2 is also added to the sample which contains A before elongation of primer 1.

3. Process for the amplification of nucleic acids A or parts thereof which comprises the following steps:
a) provision of a single-stranded nucleic acid A;
b) hybridization of the nucleic acid A with a primer P1 which contains a part which is complementary to a part of the nucleic acid A and a replication initiation site;
c) elongation of the primer P1 with a polymerase using mononucleoside triphosphates to form a nucleic acid B;
d) removal of A from the nucleic acid hybrid of A and B
e) hybridization of the nucleic acid B with a primer P2 which contains a region which is complementary to a part of the nucleic acid B and a replication initiation site;
f) elongation of the primer P2 with polymerase using mononucleoside triphosphates to form a nucleic acid C and
g) in vitro replication.

4. Method for the detection of a nucleic acid A which comprises the following steps:
a) production of a replicable nucleic acid according to the process as claimed in claim 1
b) in vitro replication
c) detection of the nucleic acids formed by replication

5. Method as claimed in claim 4, wherein the in vitro replication is carried out using detectably or/and immobilizably labelled mononucleoside triphosphates.

6. Method as claimed in claim 5, wherein a detectably labelled mononucleoside triphosphate is used, the labelled nucleic acids formed by the replication are hybridized with an immobilizably labelled nucleic acid probe and the hybrid formed is detected after binding to a solid phase.

7. Reagent kit for the production of nucleic acids, which are replicable in vitro, from a template nucleic acid A by a process as claimed in claim 1 which contains in two separate containers:
- two primers P1 and P2 which each contain nucleotide sequences which are template-specific for A and which in addition each contain a replication initiation site,
- a polymerase which is capable of elongating a primer which is hybridized with a nucleic acid using monodeoxyribonucleoside triphosphates,
- monodeoxyribonucleoside triphosphates.

8. Reagent kit for the amplification of nucleic acids A or parts thereof by a process as claimed in claim 3 which contains in separate containers:
- two primers P1 and P2 which each contain nucleotide sequences which are template-specific for A and which in addition each contain a replication initiation site,
- a polymerase which is capable of elongating a primer which is hybridized with a nucleic acid using monodeoxyribonucleoside triphosphates,
- an in vitro replication system,
- monodeoxyribonucleoside triphosphates.

9. Reagent kit for the detection of nucleic acids A by a process as claimed in claim 4 which contains in separate containers:
- two primers P1 and P2 which each contain nucleotide sequences which are template-specific for A and which in addition each contain a replication initiation site,
- a polymerase which is capable of elongating a primer which is hybridized with a nucleic acid using monodeoxyribonucleoside triphosphates,
- an in vitro replication system,
- monodeoxyribonucleoside triphosphates,
- an immobilizably labelled nucleic acid probe which is specific for nucleic acid A.

10. Reagent kit as claimed in one of the claims 7 to 9, wherein at least one of the monodeoxyribonucleoside triphosphates is detectably labelled.

## Revendications

1. Procédé de préparation d'acides nucléiques pouvant se répliquer in vitro, à partir d'une sonde nucléique A, comprenant les étapes consistant à :
a) préparer un acide nucléique A monobrin ;
b) hybrider l'acide nucléique A à une amorce P1, qui contient un fragment complémentaire d'un fragment de l'acide nucléique A et un site d'initiation de la réplication ;
c) allonger l'amorce P1 à l'aide d'une polymérase, en utilisant des mononucléosidetriphosphates, pour obtenir un acide nucléique B ;
d) éliminer A à partir de l'acide nucléique hybride de A et B ;
e) hybrider l'acide nucléique B à une amorce P2, qui contient un fragment complémentaire d'un fragment de l'acide nucléique B et un site d'initiation de la réplication, et
f) allonger l'amorce P2 à l'aide de la polymérase en utilisant des mononucléotidetriphosphates pour obtenir un acide nucléique C.

2. Procédé selon la revendication 1, caractérisé par le fait qu'avant l'allongement de l'amorce P1, on ajoute également l'amorce P2 à la sonde qui contient A.

3. Procédé d'accroissement d'acides nucléiques A ou de parties de ces acides, comprenant les étapes consistant à :
a) préparer un acide nucléique A monobrin ;
b) hybrider l'acide nucléique A à une amorce P1, qui contient un fragment complémentaire d'un fragment de l'acide nucléique A et un site d'initiation de la réplication ;
c) allonger l'amorce P1 à l'aide d'une polymérase, en utilisant des mononucléosidetriphosphates, pour obtenir un acide nucléique B ;
d) éliminer A à partir de l'acide nucléique hybride de A et B ;
e) hybrider l'acide nucléique B à une amorce P2, qui contient un fragment complémentaire d'un fragment de l'acide nucléique B et un site d'initiation de la réplication ;
f) allonger l'amorce P2 à l'aide de la polymérase en utilisant des mononucléotidetriphosphates pour obtenir un acide nucléique C ; et
g) effectuer une réplication in vitro.

4. Procédé de détection d'un acide nucléique A, qui comprend les étapes consistant à :
a) préparer un acide nucléique pouvant se répliquer selon le procédé de la revendication 1,
b) effectuer une réplication in vitro,
c) détecter l'acide nucléique formé par la réplication.

5. Procédé selon la revendication 4, caractérisé par le fait que la réplication in vitro est réalisée en utilisant des mononucléosidetriphosphates marqués détectables et/ou immobilisables.

6. Procédé selon la revendication 5, caractérisé par le fait que l'on met en oeuvre un mononucléosidetriphosphate marqué détectable, que les acides nucléiques marqués formés par la réplication sont hybridés à une sonde nucléique marquée immobilisable et que l'hybride formé est détecté après fixation sur une phase solide.

7. Kit de réactifs permettant la préparation d'acides nucléiques pouvant se répliquer in vitro, à partir d'une sonde nucléique A, par un procédé selon la revendication 1, contenant, dans des récipients séparés :
- deux amorces P1 et P2, qui contiennent chacune des séquences nucléotidiques qui sont spécifiques à la sonde A et qui contiennent en outre chacune un site d'initiation de la réplication,
- une polymérase, qui est capable d'allonger une amorce hybridée à un acide nucléique, avec des monodésoxyribonucléosidetriphosphates,
- des monodésoxyribonucléosidetriphosphates.

8. Kit de réactifs permettant d'allonger des acides nucléiques A ou des parties de ces acides par un procédé selon la revendication 3, contenant, dans des récipients séparés :
- deux amorces P1 et P2, qui contiennent chacune des séquences nucléotidiques qui sont spécifiques à la sonde A et qui contiennent en outre chacune un site d'initiation de la réplication,
- une polymérase, qui est capable d'allonger une amorce hybridée à un acide nucléique, avec des monodésoxyribonucléosidetriphosphates,
- un système de réplication in vitro,
- des monodésoxyribonucléosidetriphosphates.

9. Kit de réactifs permettant la détection d'acides nucléiques A par un procédé selon la revendication 4, contenant, dans des récipients séparés :
- deux amorces P1 et P2, qui contiennent chacune des séquences nucléotidiques qui sont spécifiques à la sonde A et qui contiennent en outre chacune un site d'initiation de la réplication,
- une polymérase, qui est capable d'allonger une amorce hybridée à un acide nucléique, avec des monodésoxyribonucléosidetriphosphates,
- un système de réplication in vitro,
- des monodésoxyribonucléosidetriphosphates,
- une sonde nucléique marquée immobilisable, spécifique à un acide nucléique A.

10. Kit de réactifs selon l'une quelconque des revendications 7 à 9, caractérisé par le fait qu'au moins un des monodésoxyribonucléoside-triphosphates est marqué de façon détectable.
